# EUROPÄISCHE PATENTSCHRIFT

(11) **EP 0 884 066 B1**
(45) Veröffentlichungstag und Bekanntmachung des Hinweises auf die Patenterteilung: **19.10.2005**
(21) Anmeldenummer: 98110195.9
(22) Anmeldetag: 04.06.1998
(51) Int. Cl.: A61N 5/06

(54) **Vorrichtung zur kosmetischen Behandlung von Akne Vulgaris**
Device of cosmetical treatment of vulgaris acne
Dispostif de soins cosmétiques de l'acné vulgaris

(30) Priorität: 09.06.1997 DE 19724299
(43) Veröffentlichungstag der Anmeldung: 16.12.1998
(73) Patentinhaber: SLI Lichtsysteme GmbH, 91056 Erlangen (DE)
(72) Erfinder: Köhler, Wolfgang, 91220 Schnaittach (DE)
(74) Vertreter: Lemke, Jörg-Michael

(56) Entgegenhaltungen:
- WO-A-96/14899
- DE-A- 4 026 327
- DE-A- 4 143 168
- GB-A- 2 190 994
- US-A- 5 549 660

## Beschreibung

Die Erfindung betrifft eine Vorrichtung zur Behandlung von Akne Vulgaris durch Bestrahlung der betreffenden Partien mit Licht.

Es ist eine Vorrichtung dieser Art bekannt, bei welchem zur Bestrahlung speziell des Gesichts UV-Licht verwendet wird. Dabei ist jedoch nachteiligerweise eine Erythembildung möglich, ferner eine unerwünschte Oxidation der Hautpigmente. WO-A-96/14899 offenbart eine Vorrichtung zur Bestrahlung von Hautbereichen mittels einer Leuchte mit einem Gehäuse, in dem zumindest eine Lampe und ein Reflektor dahinter angeordnet sind. Die bekannte Vorrichtung kann zwei Leuchtstoffe aufweisen mit jeweils einem ersten Emissionsspektrum im blauen und jeweils einem zweiten Emissionsspektrum im roten Bereich. Die Vorrichtung ist zur diagnostischen Nutzung vorgesehen.

Ferner ist eine Behandlung mit Creme bekannt, die etwa 0,5 % Benzoylperoxid enthält. Der Nachteil dieser Behandlung besteht in Hautaustrocknung.

Die der Erfindung zugrundeliegende Aufgabe wird darin gesehen, eine Vorrichtung der eingangs genannten Art zu schaffen, wodurch nicht nur die genannten Nachteile vermieden werden, sondern darüberhinaus sich ein besonders guter kosmetischer Effekt erzielen läßt.

Diese Aufgabe wird nach der Erfindung dadurch gelöst, daß Licht mit zwei Emissionsspektren verwendet wird, von denen das eine im blauen Bereich von 400 nm bis 450 nm und das andere im roten Bereich von 580 nm bis 630 nm liegt.

Die Bestrahlung mit der erfindungsgemäßen Vorrichtung ergibt weder UV-Schäden noch signifikante Hautaustrocknung.

Erfindungsgemäß addieren sich die beiden Emissionsspektren zu einem Gesamtspektrum, das dem Aktionsspektrum der Inaktivierung des Propionibacterium acnes angepaßt ist und mit einem biostimulierenden Effekt auf die Zellen der Haut wirkt. Das Propionibacterium acne enthält nämlich Porphyrine, die durch Licht großer Wellenlänge erregt werden können, was zum Tod des Bacteriums führt.

Es wurde eine erfindungsgemäße Vorrichtung eingesetzt und eine Bestrahlung mit die genannten Emissionsspektren aufweisendem, blaurotem Mischlicht mit einer Bestrahlung mit blauem Licht und mit einer Bestrahlung mit weißem Licht verglichen, ferner auch mit einer Behandlung mit der genannten Benzoylperoxid-Creme, und zwar an 61 Probanden, die leichte bis mittlere Akne aufwiesen.

Die Probanden waren instruiert worden, jeden Tag eine Bestrahlung von 15 Minuten Länge mit entsprechenden Leuchten durchzuführen, bzw. die genannte Creme zweimal täglich aufzutragen.

Die Auswertung an den Probanden erfolgte alle vier Wochen. Die folgende Tabelle zeigt das Ergebnis dieser Auswertung:

| **Beobachtung** | **Blau / rotes Licht** | | **Blaues Licht** | | **Weißes Licht** | | **Creme** | |
|---|---|---|---|---|---|---|---|---|
| | Arzt % | Patient % | Arzt % | Patient % | Arzt % | Patient % | Arzt % | Patient % |
| Schlechter / Unverändert | 27 | 27 | 25 | 50 | 46 | 46 | 19 | 19 |
| Leichte / bescheidene Besserung | 18 | 27 | 42 | 33 | 46 | 46 | 44 | 50 |
| markante Besserung | 55 | 46 | 33 | 17 | 8 | 8 | 37 | 31 |

Die unter "Arzt" aufgeführte Zahl entspricht jeweils der Beurteilung durch einen Arzt, die unter "Patient" der des Probanden nach der Bestrahlungs- bzw. Cremekur.

Wie man der Tabelle entnimmt, wurden die besten Resultate mit dem erfindungsgemäßen blauroten Mischlicht erzielt, und zwar mit einer mittleren Reduktionsrate von 66 % bei entzündlichen und 42 % bei nichtentzündlichen Läsionen, während blaues Licht diesbezüglich 50 % und 32 %, weißes Licht 21 % und 0 %, und Benzoylperoxid-Creme 61 % und 58 % ergab.

Die ärztliche Auswertung ergab eine markante Besserung bei 55 % der Fälle unter Verwendung des erfindungsgemäßen rotblauen Mischlichts, bei 33 % der Fälle bei blauem Licht, bei 8 % der Fälle bei weißem Licht und bei 37 % der Fälle bei Verwendung der Benzoylperoxid-Creme.

Die Auswertung durch die Probanden selber zeigte jeweils markante Verbesserungen mit 46 % bei Verwendung des erfindungsgemäßen rotblauen Mischlichts, von 16 % bei blauem Licht, von 8 % bei weißem Licht und von 31 % bei Cremebehandlung. Trockenheit der Haut wurde speziell bei der Cremebehandlung festgestellt. Bei der Lichtbehandlung war sie demgegenüber geringfügig.

Figur 1 zeigt die spektrale Energieverteilung einer Lampe mit blauem Leuchtstoff, während Figur 2 dieselbe bei einer Leuchtstofflampe mit rotem Leuchtstoff darstellt. Wie man feststellt, befinden sich die erfindungsgemäßen Emissionsspektren jeweils innerhalb des Bereichs der hauptsächlichen spektralen Energie.

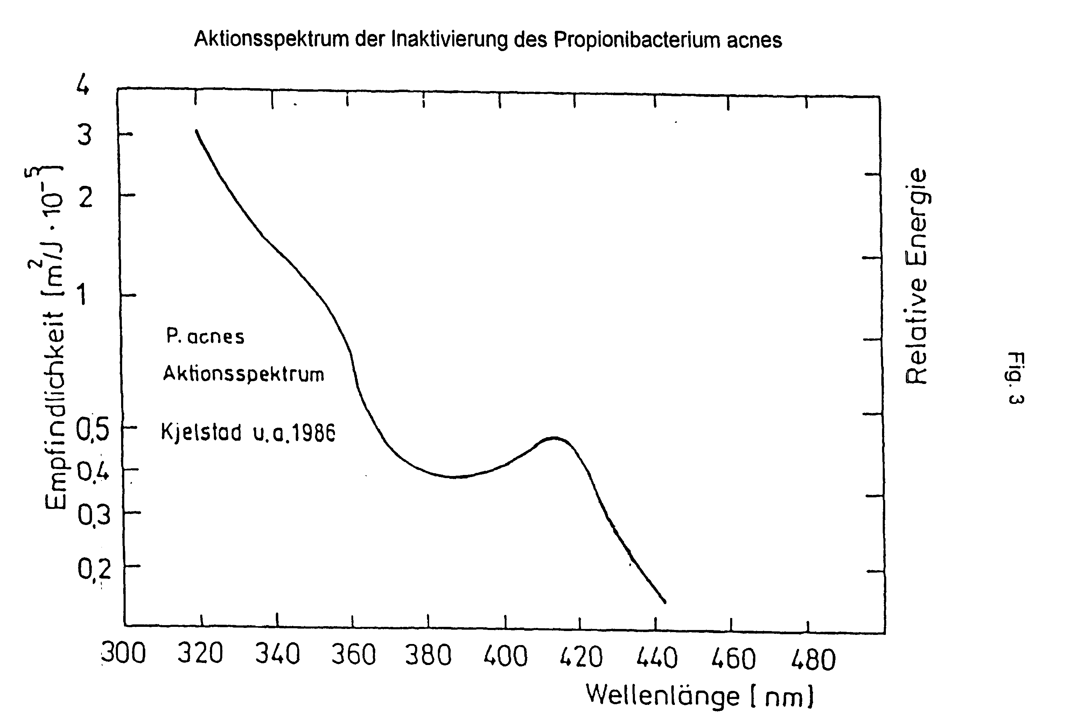

Figur 3 zeigt das Aktionsspektrum der Inaktivierung des Propionibacterium acnes.

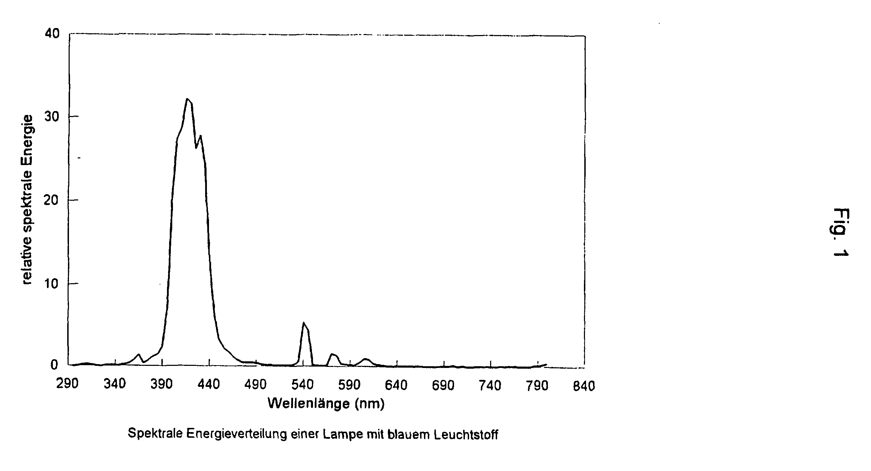

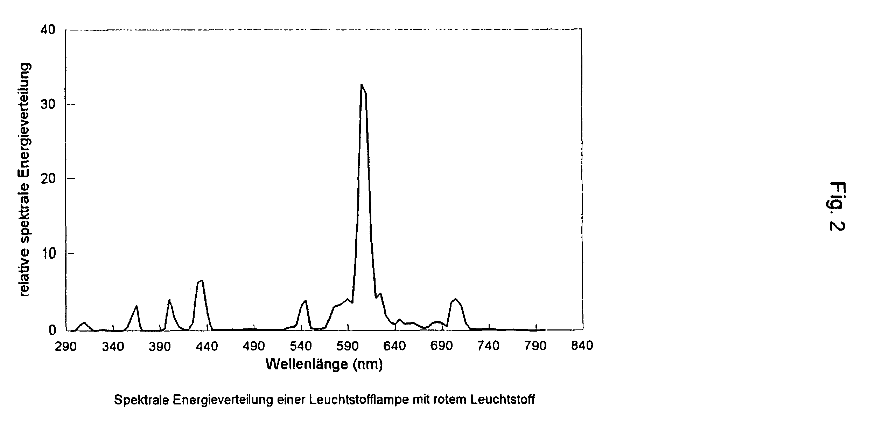

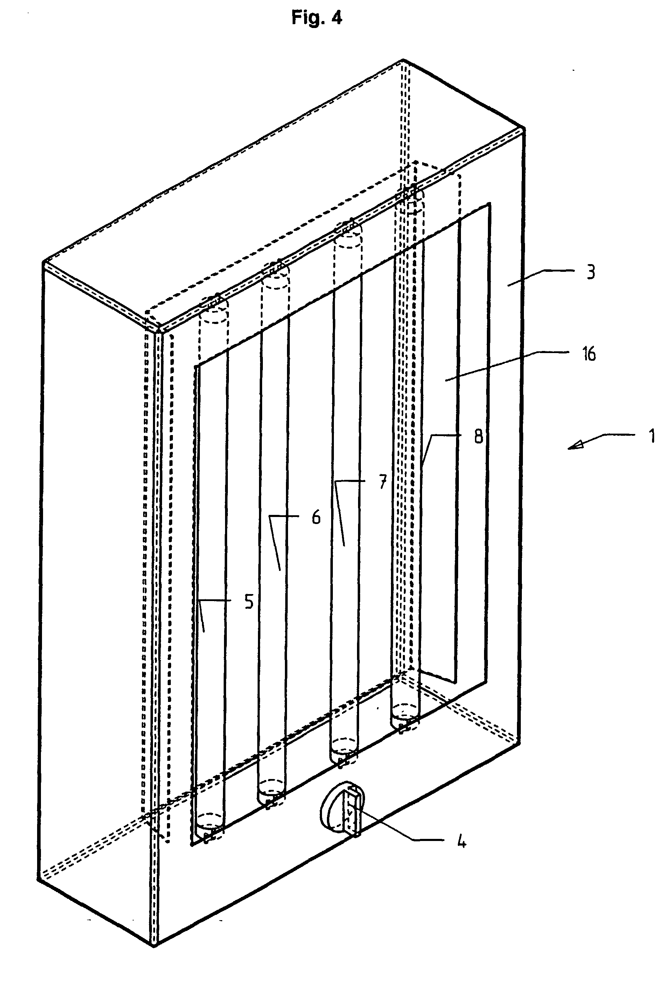

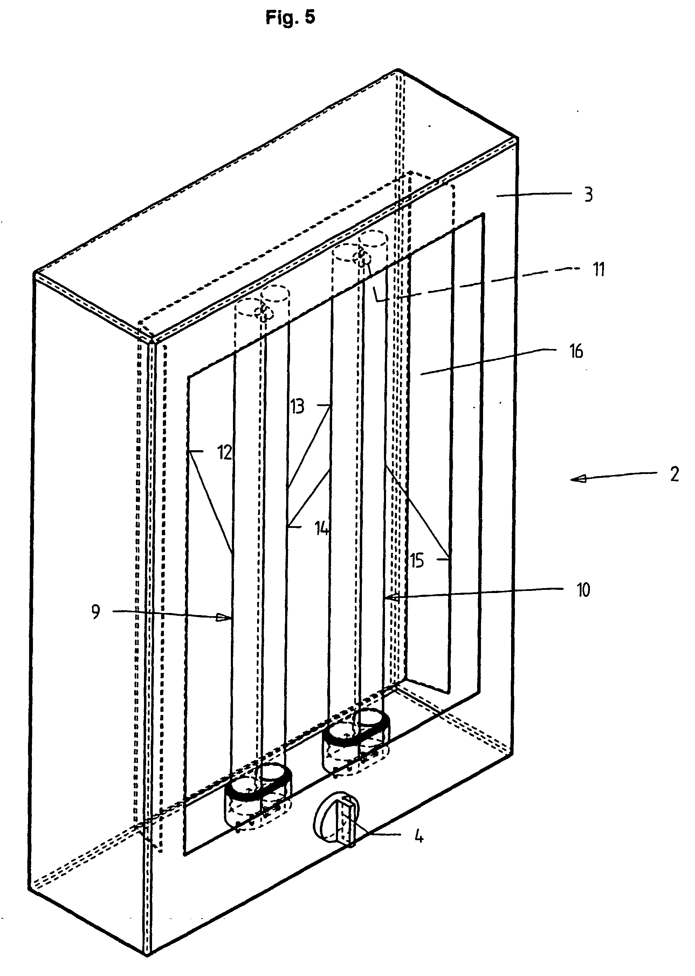

Im folgenden werden anhand der Fig. 4 und 5 bevorzugte Ausführungsformen von Lampen beschrieben, deren Emissionsspektren den Diagrammen nach den Fig. 1 und 2 entsprechen, so daß sich mit denselben die beschriebenen kosmetischen Behandlungen mit blaurotem Licht nach der Erfindung durchführen lassen. Mit einer Leuchte entsprechend der Fig. 4 wurden die Versuche durchgeführt und die in obiger Tabelle unter "Blau/rotes Licht" angegebenen Werte ermittelt.

Beide erfindungsgemäßen Leuchten 1 bzw. 2 besitzen ein hier rechteckiges Gehäuse 3, in dem zumindest eine Lampe und zumindest ein Reflektor dahinter angeordnet sind. Ferner ist im unteren Bereich des Gehäuses 3 eine Schaltuhr 4 zur Einstellung und Begrenzung der Bestrahlungszeit vorgesehen.

Bei der in Fig. 4 gezeigten, ersten Ausführungsform sind vier Lampen vorgesehen, und zwar jeweils zweiseitig gesockelte und im wesentlichen parallel zueinander angeordnete Leuchtstofflampen 5, 6, 7 und 8 mit einem Kolbendurchmesser von wahlweise 16 mm bis 38 mm und einer Länge von wahlweise 300 mm bis 600 mm. Zwei von ihnen emittieren im blauen Bereich und zwei im roten Bereich, wobei die Anordnung so getroffen ist, daß die Leuchtstofflampen 5 und 7 im blauen und die Leuchtstofflampen 6 und 8 im roten Bereich emittieren, derart, daß sich in der Lampenanordnung vorzugsweise jeweils eine blaue und eine rote Lampe abwechseln.

Bei der in Figur 5 gezeigten Ausführungsform sind in der Leuchte 2 zwei einseitig gesockelte Leuchtstofflampen 9 und 10 nach DIN EN 60901 mit Sockeln 2G11 und mit zwei durch einen Steg 11 miteinander verbundenen Schenkeln 12, 13 bzw. 14, 15 mit einer Gesamtlänge von wahlweise 225 mm bis 415 mm angeordnet, wobei jeweils der eine Schenkel 12 bzw. 14 einer Lampe 9 bzw. 10 im erfindungsgemäßen blauen Bereich und der andere Schenkel 13 bzw. 15 derselben im erfindungsgemäßen roten Bereich emittiert.

Beide Lampen 1 und 2 besitzen jeweils einen einzigen, gleichartig ausgebildeten Reflektor 16.

## Patentansprüche

1. Vorrichtung zur Bestrahlung von Akne vulgaris aufweisenden Hautbereichen mittels einer Leuchte (1, 2) mit einem Gehäuse (3), in dem zumindest eine Lampe (5, 6, 7, 8; 9, 10) und zumindest ein Reflektor (16) dahinter angeordnet sind, mit zwei Leuchtstoffen mit jeweils einem ersten Emissionsspektrum im blauen Bereich von 400 nm bis 450 nm und jeweils einem zweiten Emissionsspektrum im roten Bereich von 580 nm bis 630 nm, sowie mit einer Schaltuhr (4) zur Einstellung und Begrenzung der Bestrahlungszeit.

2. Vorrichtung nach Anspruch 1, **dadurch gekennzeichnet, dass** die Leuchte (1, 2) vier zweiseitig gesockelte und im wesentlichen parallel zueinander angeordnete Leuchtstofflampen (5, 6, 7, 8) mit einem Kolbendurchmesser von 16 mm bis 38 mm und einer Länge von 300 mm bis 600 mm aufweist, von denen zwei (5, 7) in dem genannten blauen Bereich und zwei in dem genannten roten Bereich (6, 8) emittieren.

3. Vorrichtung nach Anspruch 2, **dadurch gekennzeichnet, daß** sich in der Anordnung innerhalb der Leuchte (1) jeweils eine blau strahlende (5, 7) und eine rot strahlende (6, 8) Lampe abwechseln.

4. Vorrichtung nach Anspruch 1, **dadurch gekennzeichnet, daß** in der Leuchte (2) zwei einseitig gesockelte Leuchtstofflampen (9, 10) nach DIN EN 60901 mit Sockeln 2G11 und mit zwei durch einen Steg (11) miteinander verbundenen Schenkeln (12, 13; 14, 15) mit einer Gesamtlange von 225 mm bis 415 mm vorgesehen sind, wobei jeweils der eine Schenkel (12, 14) einer Lampe (9, 10) im blauen Bereich und der andere Schenkel 813, 15) derselben im roten Bereich emittiert.

## Claims

1. Apparatus for the irradiation of skin showing Akne vulgaris by a luminaire (1, 2) having a housing (3) in which at least one lamp (5, 6, 7, 8; 9, 10) and at least one reflector (16) behind are arranged, with two fluorescents, the one having a first emission spectrum in the blue region from 400 nm to 450 nm, and the other having a second emission spectrum in the red region from 580 nm to 630 nm, and with a switch clock (4) for the adjustment and limitation of the irradiation time.

2. Apparatus according to claim 1, **characterized in that** the luminaire (1, 2) has four two ended and based fluorescent lamps (5, 6, 7, 8) arranged parallel to each other, having a bulb diameter from 16mm to 38mm and a lenth from 300mm to 600mm, two of those (5, 7) emitting in the said blue region and two of those (6, 8) emitting in the said red region.

3. Apparatus according to claim 2, **characterized in that** within the arrangement in the luminaire (1) a blue emitting (5, 7) and a red emitting (6, 8) alternate respectively.

4. Apparatus according to claim 1, **characterized in that** in the luminaire (2) two one ended and based fluorescent lamps (9, 10) following DIN EN 60901 with bases 2G11 and two legs (12, 13; 14, 15) connected with each other by a bridge (11) are provided having an overall length from 225mm to 415mm, wherein the one leg (12, 14) of a lamp (9, 10) emits in the blue region and the other leg (13, 15) emits in the red region.

## Revendications

1. Dispositif d'exposition à un rayonnement de parties de la peau présentant de l'acné vulgaris au moyen d'un appareil (1, 2) d'éclairage ayant un boîtier (3), dans lequel sont montés au moins une lampe (5, 6, 7, 8, 9, 10) et au moins un réflecteur (16) derrière, comprenant deux substances luminescentes ayant, respectivement, un premier spectre d'émission dans le domaine bleu allant de 400 nm à 450 nm et, respectivement, un deuxième spectre d'émission dans le domaine rouge allant de 580 nm à 630 nm, ainsi qu'une minuterie (4) pour régler et limiter la durée d'exposition.

2. Dispositif suivant la revendication 1, **caractérisé en ce que** l'appareil (1, 2) d'éclairage a quatre lampes (5, 6, 7, 8) fluorescentes à culot des deux côtés et disposées sensiblement parallèlement les unes aux autres ayant un diamètre d'ampoule de 16 mm et 38 mm et une longueur de 300 mm à 600 mm, dont deux (5, 7) émettent dans ledit domaine bleu et deux (6, 8) dans ledit domaine rouge.

3. Dispositif suivant la revendication 2, **caractérisé en ce que**, dans l'agencement à l'intérieur de l'appareil (1) d'éclairage, respectivement, une lampe (5, 7) émettant dans le bleu alterne avec une lampe (6, 8) émettant dans le rouge.

4. Dispositif suivant la revendication 1, **caractérisé en ce que**, dans l'appareil (2) d'éclairage, sont prévues deux lampes (9, 10) fluorescentes à culot d'un côté suivant la norme DIN EN 60901 ayant des culots 2G11 et ayant deux branches (12, 13, 14, 15) reliées entre elles par une âme (11) d'une longueur totale de 225 mm à 415 mm, respectivement l'une des branches (12, 14) d'une lampe (9, 10) émettant dans le domaine bleu et l'autre branche (13, 15) de celle-ci dans le domaine rouge.
